# EUROPEAN PATENT APPLICATION

(11) **EP 4 198 990 A1**
(43) Date of publication of application: **21.06.2023**
(21) Application number: 22742047.8
(22) Date of filing: 12.01.2022
(51) Int. Cl.: G16B 15/20

(54) **DATA PROCESSING METHOD AND APPARATUS, AND COMPUTER DEVICE AND STORAGE MEDIUM**

(30) Priority: 19.01.2021 CN 202110065836
(71) Applicant: Tencent Technology (Shenzhen) Company Limited, Shenzhen, Guangdong 518057 (CN)
(72) Inventor: PEI, Jianguo, Shenzhen, Guangdong 518057 (CN); LIU, Wei, Shenzhen, Guangdong 518057 (CN); HUANG, Junzhou, Shenzhen, Guangdong 518057 (CN)
(74) Representative: Eisenführ Speiser
(86) International application number: PCT/CN2022/071490
(87) International publication number: WO 2022/156568

(57) **Abstract**

A data processing method and apparatus, and a computer device and a storage medium. The method comprises: acquiring protein attribute information of a reference protein substance, wherein the reference protein substance comprises a protein adjustment area (S 101); by means of a protein prediction model and according to the protein attribute information, generating a predicted protein fragment at the protein adjustment area in the reference protein substance, wherein the protein prediction model is obtained by means of training on the basis of a target protein substance, and the protein prediction model is used for predicting a protein substance that is combined with the target protein substance (S 102); matching, in a protein fragment database, a similar protein fragment of the predicted protein fragment (S 103); and performing virtual synthesis on the similar protein fragment and the reference protein substance, so as to obtain synthetic substance auxiliary information, wherein the synthetic substance auxiliary information is used for assisting in generating an antibody protein substance combined with the target protein substance (S 104). By means of the method, the efficiency of acquiring an antibody protein substance can be improved.

## Description

This application claims priority to Chinese Patent Application No. 202110065836X, filed with the Chinese Patent Office on January 19, 2021 and entitled "DATA PROCESSING METHOD AND APPARATUS, COMPUTER DEVICE, AND STORAGE MEDIUM", which is incorporated herein by reference in its entirety.

### FIELD OF THE TECHNOLOGY

The present disclosure relates to the technical field of artificial intelligence, and in particular to a data processing technology.

### BACKGROUND OF THE DISCLOSURE

A target protein is a pathogenic protein. An antibody protein for binding to the target protein is normally designed by modifying a reference antibody protein originally existing in a human body. The modified reference antibody protein is the antibody protein for binding to the target protein.

For specific implementation, the reference antibody protein may be modified by using a short chain polypeptide from a short chain polypeptide database, where the short chain polypeptide database generally includes a plurality of short chain polypeptides collected from nature, and each short chain polypeptide is a protein fragment. In the related art, generally the short chain polypeptide may be randomly selected from the short chain polypeptide database continuously, to modify the reference antibody protein. Each time when the reference antibody protein is modified by using one short chain polypeptide, it is evaluated whether the modified reference antibody protein meets the standard of binding to the target protein. The selection of the short chain polypeptides from the short chain polypeptide database to modify the reference antibody protein is repeated until the modified reference antibody protein is evaluated as meeting the standard of binding to the target protein.

Therefore, in the related art, the selecting the short chain polypeptides for modifying the reference antibody protein is very random, so that the reference antibody protein normally needs to be modified by a large amount of short chain polypeptides in order to obtain the reference antibody protein meeting the standard of binding to the target protein, resulting in low efficiency of acquiring the antibody protein for binding to the target protein.

### SUMMARY

The present disclosure provides a data processing method and apparatus, a computer device and a storage medium, which can improve the efficiency of acquiring antibody protein.

An aspect of the present disclosure provides a data processing method, performed by a computer device, the method including:
acquiring protein attribute information of a reference protein substance, the reference protein substance including a protein adjusting region;
generating, by using a protein prediction model, a predicted protein fragment at the protein adjusting region in the reference protein substance according to the protein attribute information, the protein prediction model being obtained by training based on a target protein substance; the protein prediction model being configured to predict a protein substance for binding to the target protein substance;
identifying a similar protein fragment matching the predicted protein fragment from a protein fragment database;
performing virtual synthesis on the similar protein fragment and the reference protein substance to obtain synthetic substance auxiliary information; and the synthetic substance auxiliary information being used for assisting in generation of an antibody protein substance for binding to the target protein substance.

Another aspect of the present disclosure provides a data processing apparatus, including:
an attribute acquiring module, configured to acquire the protein attribute information of the reference protein substance, the reference protein substance including a protein adjusting region;
a predicted fragment generating module, configured to generate, by using the protein prediction model, a predicted protein fragment at the protein adjusting region in the reference protein substance according to the protein attribute information, the protein prediction model being obtained by training based on the target protein substance, the protein prediction model being configured to predict a protein substance for binding to the target protein substance;
a fragment matching module, configured to identify a similar protein fragment matching the predicted protein fragment from a protein fragment database;
a substance synthesizing module, configured to perform virtual synthesis on the similar protein fragment and the reference protein substance to obtain synthetic substance auxiliary information, the synthetic substance auxiliary information being used for assisting in generation of an antibody protein substance for binding to the target protein substance.

An aspect of the present disclosure provides a computer device, including a memory and a processor, the memory storing a computer program; and the computer program, when executed by the processor, causing the processor to perform the method according to the aspect of the present disclosure.

An aspect of the present disclosure provides a computer-readable storage medium, the computer-readable storage medium storing a computer program, the computer program including a program instruction, the program instruction, when executed by a processor, causing the processor to perform the foregoing method according to the foregoing aspect.

An aspect of the present disclosure provides a computer program product or a computer program, the computer program product or the computer program including computer instructions, the computer instructions being stored in a computer-readable storage medium. A processor of a computer device reads the computer instructions from the computer-readable storage medium, and executes the computer instructions, so that the computer device performs the method provided in the various optional implementations in the foregoing aspect.

In the present disclosure, the protein attribute information of the reference protein substance is acquired. The reference protein substance including a protein adjusting region; generating a predicted protein fragment at the protein adjusting region in the reference protein substance according to the protein attribute information by using a protein prediction model. The protein prediction model is obtained by training on the basis of a target protein substance, and is configured to predict a protein substance for binding to the target protein substance. The similar protein fragment matching the predicted protein fragment is identified from a protein fragment database. Virtual synthesis is performed on the similar protein fragment and the reference protein substance to obtain synthetic substance auxiliary information, The synthetic substance auxiliary information is used for assisting in generation of an antibody protein substance for binding to the target protein substance. Therefore, according to the method provided by the present disclosure, on the basis of the predicted protein fragment predicted by the protein prediction model, the similar protein fragment used to modify the protein fragment at the protein adjusting region in the reference protein substance can be rapidly determined, and the antibody protein substance for binding to the target protein substance can be rapidly generated on the basis of the similar protein fragment, thereby improving the efficiency of acquiring the antibody protein substance.

### BRIEF DESCRIPTION OF THE DRAWINGS

FIG. 1 is a schematic structural diagram of a network architecture according to an embodiment of the present disclosure.
FIG. 2 is a schematic diagram of a scenario of data prediction according to the present disclosure.
FIG. 3 is a schematic flowchart of a data processing method according to the present disclosure.
FIG. 4 is a schematic diagram of a scenario of fragment prediction according to the present disclosure.
FIG. 5 is a schematic diagram of a scenario of fragment generation according to the present disclosure.
FIG. 6 is a schematic diagram of a scenario of model training according to the present disclosure.
FIG. 7 is a schematic diagram of a scenario of synthesis of an antibody protein substance according to the present disclosure.
FIG. 8 is a schematic diagram of a scenario of data interaction according to the present disclosure.
FIG. 9 is a schematic structural diagram of a data processing apparatus according to the present disclosure.
FIG. 10 is a schematic structural diagram of a computer device according to the present disclosure.

### DESCRIPTION OF EMBODIMENTS

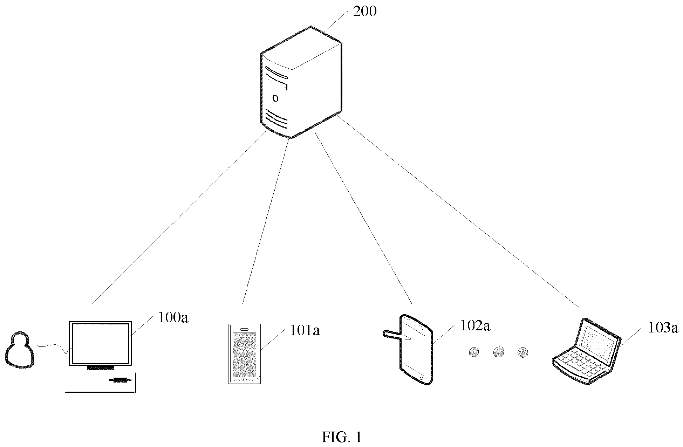

FIG. 1 is a schematic structural diagram of a network architecture according to an embodiment of the present disclosure. As shown in FIG. 1, the network architecture may include a server 200 and a terminal device cluster. The terminal device cluster may include one or more terminal devices, the number of which is not limited here. As shown in FIG. 1, the plurality of terminal devices may specifically include a terminal device 100a, a terminal device 101a, a terminal device 102a...and a terminal device 103a. As shown in FIG. 1, the terminal device 100a, the terminal device 101a, the terminal device 102a...and the terminal device 103a may all be in network connection with the server 200, so that each terminal device is in data interaction with the server 200 by the network connection.

The server 200 shown in FIG. 1 may be an independent physical server, or may be a server cluster or distributed system including a plurality of physical servers, or may be a cloud server providing basic cloud computing services, such as a cloud service, a cloud database, cloud computing, a cloud function, cloud storage, a network service, cloud communication, a middleware service, a domain name service, a security service, a content delivery network (CDN), big data an artificial intelligence platform. The terminal devices may be an intelligent terminal such as a smart phone, a tablet computer, a notebook computer, a desktop computer and an intelligent television. The following takes communication between the terminal device 100a and the server 200 as an example to specifically describe the embodiment of the present disclosure.

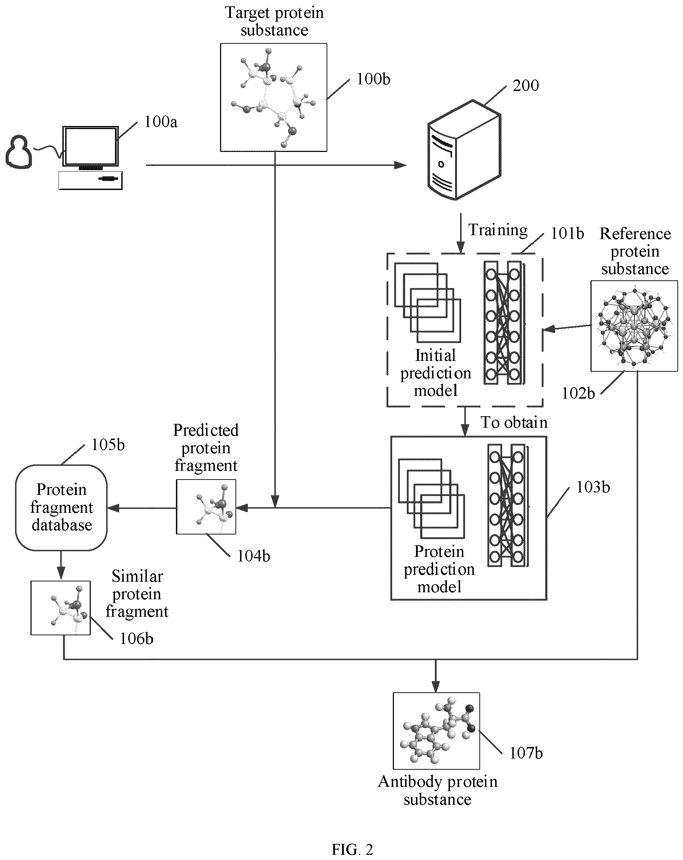

FIG. 2 is a schematic diagram of a scenario of data prediction according to the present disclosure. Referring to FIG. 2, the terminal device 100a may be a terminal device used for a pharmaceutical-factory. The terminal device 100a may provide a target protein substance 100b for the server 200. For example, the terminal device 100a may transmit related description information of the target protein substance 100b to the server 200. The related description information of the target protein substance 100b is description information used for determining the target protein substance 100b uniquely, for example, the related description information may be structure information, torsion angle information, protein sequence information and the like of the target protein substance 100b. The target protein substance 100b is a pathogenic protein, for example, the target protein substance 100b may be a cancer protein in a human body.

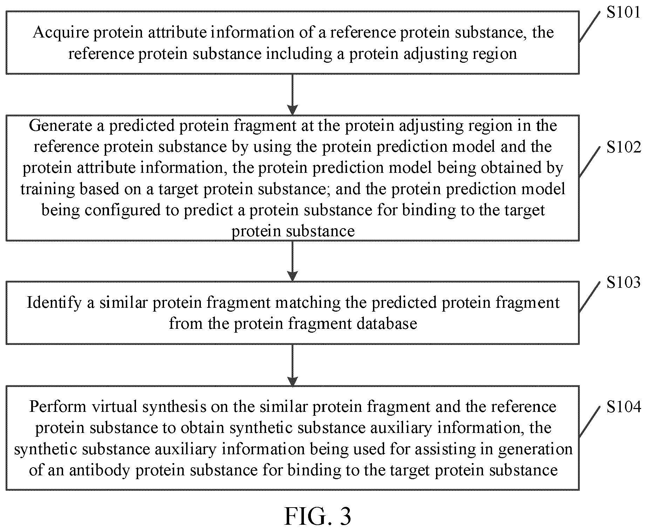

After the server 200 acquires the target protein substance 100b provided by the terminal device 100a, the server 200 may train the initial prediction model 101b on the basis of the target protein substance 100b and the reference protein substance 102b jointly so as to obtain the protein prediction model 103b. The reference protein substance 102b is a protein substance existing in a human body and capable of binding to the target protein substance 100b after being modified. The protein prediction model 103b obtained by training the initial prediction model 101b is configured to predict a protein substance capable of binding to the target protein substance 100b. By predicting the protein substance for binding to the target protein substance, an antibody protein substance configured to treat a disease (such as cancer) relating to the target protein substance 100b. The specific process of training the initial prediction model 101b to obtain the protein prediction model 103b is described below with reference to an embodiment of FIG. 3.

The reference protein substance 102b may include a protein adjusting region, where the protein adjusting region is a variable region, that is, a protein fragment at the protein adjusting region in the reference protein substance 102b is modifiable. A protein fragment 104b for the reference protein substance 102b at the protein adjusting region may be generated by using the protein prediction model 103b. The protein fragment 104b may be referred to as a predicted protein fragment, which is the modified protein fragment of the reference protein substance 102b at the protein adjusting region.

A protein fragment database 105b may include a plurality of protein fragments collected from the nature, and the server 200 may search the protein fragment database 105b for a similar protein fragment 106b to the predicted protein fragment 104b. Then, the server 200 may synthesize the similar protein fragment 106b and the reference antibody protein 102b, to obtain an antibody protein substance 107b. The antibody protein substance is configured to bind to the target protein substance 100b to achieve the purpose of treating a disease (such as cancer) relating to the target protein substance 100b. The specific process of acquiring the antibody protein substance 107b is described below with reference to the embodiment corresponding to FIG. 3.

By the method provided by the present disclosure, AI is applied to a medical pharmacy scenario for assistance, to improve the efficiency of obtaining a similar protein fragment, and improve the efficiency of acquiring an antibody protein substance for a target protein substance. Therefore, the method provided by the present disclosure can save the medical pharmacy cost and improve the medical pharmacy speed.

FIG. 3 is a schematic flowchart of a data processing method according to the present disclosure. As shown in FIG. 3, the method may include steps S101 to S104.

Step S101: Acquire protein attribute information of a reference protein substance, the reference protein substance including a protein adjusting region.

Specifically, the execution subject of the embodiment of the present disclosure may be one computer device or a computer device cluster composed of a plurality of computer devices. The computer device may be a server, and may also be a terminal device. Therefore, the method provided by this embodiment of the present disclosure may be performed by the server, or may be performed by the terminal device, or may be performed by both the server and the terminal device. The following description of embodiments of the present disclosure is made with an example that the execution subject is a server.

The reference protein substance is a certain specific protein which exists in a human body and can be modified. A macromolecular antibody protein may be obtained by modifying the reference protein substance. The macromolecular antibody protein is a protein for treating a disease. The macromolecular antibody protein may bind to a pathogenic protein to achieve the effect of treating the disease. The reference antibody protein may be a TCL protein (human recombinant protein) existing in a human body.

In general, the reference protein substance may include a plurality of (at least two) amino acids. The server may acquire amino acid structure information of each amino acid included in the reference protein substance and acquire amino acid torsion angle information of each amino acid included in the reference protein substance.

The amino acid structure information of amino acids may be secondary structure information of the amino acids, which has four types including α-helix, β-sheet, β-turn and random coil, and correspondingly, the amino acid structure information of the amino acids may be the secondary structure information of the amino acids in the α-helix, β-sheet, β-turn and random coil. The secondary structure information of the amino acids is the secondary structure information of a protein substance to which the amino acids belong. The secondary structure information is specific conformation formed by coiling or folding polypeptide main chain skeleton atoms in the protein substance along a certain axis, namely the spatial position arrangement of the main chain skeleton atoms of a peptide chain.

The amino acid torsion angle information of the amino acids is the torsion angle information of the reference protein substance. The torsion angle information of a protein substance includes a torsion angle of the protein substance, which is an angle formed by crossing other bonds on adjacent carbons when a single bond in the protein substance rotates. The amino acid torsion angle information of one amino acid may include 3 angles, which are Phi angle, Psi angle and Omega angle respectively. Specifically, the phi angle is an angle rotated around a bond N-Ca (a chemical bond), the psi angle is an angle rotated around a bond Ca-C (a chemical bond), and the omega angle is an angle rotated around a C-N bond (a chemical bond).

An amino acid in the reference protein substance may have one piece of amino acid structure information and one piece of amino acid torsion angle information. The server may acquire the amino acid structure information and the amino acid torsion angle information of all amino acids in the reference protein substance as protein attribute information of the reference protein substance. In addition, the protein attribute information may further include a protein sequence (which may be simply referred to as proteins) of the reference protein substance. The protein sequence is a computer representation of the reference protein substance, that is, a machine language for the reference protein substance. The amino acid structure information and the amino terminal torsion angle information of the amino acids included in the reference protein substance can represent the structure information and the torsion angle information of the reference protein substance.

The reference protein substance is a reference antibody protein. The reference protein substance may include a variable region, such as a complementarity-determining region (CDR), i.e., a modifiable region in the reference protein substance. A protein at a diseased portion in a pathogenic protein may be referred to as a target protein. The target protein is a protein, and may also be referred to as a target protein substance. The target protein substance is a protein which is diseased in a human body. The generated macromolecular antibody protein is used to bind to the target protein substance so as to achieve the purpose of treating human diseases.

It is understood that there are generally different target protein substances for different diseases, and that different diseases may generally correspond to different variable regions (namely modification regions) in the reference protein substance. In other words, different target protein substances may correspond to the same reference protein substance, but correspond to different variable regions in the same reference protein substance.

In this embodiment of the present disclosure, the target protein substance to be bound to the generated macromolecular antibody protein may be any paraprotein in a human body, and the specific disease relating to the target protein substance may be determined according to an actual application scenario, which is not limited herein. A process of generating the macromolecular antibody protein of the target protein substance is described herein as an example.

The target protein substance may be provided by a third-party device, and the third-party device may be used for a pharmaceutical factory. When a pharmaceutical factory needs to generate a macromolecular antibody protein for binding to a certain target protein substance, the pharmaceutical factory may provide the target protein substance to the server by using the third-party device. For example, the pharmaceutical factory may transmit the related description information of the target protein substance to the server by using the third-party device. The related description information is information for describing and determining the target protein substance, which may for example, include protein sequence information and protein structure information of the target protein substance. Alternatively, the pharmaceutical factory may provide a pdb file of the target protein substance to the server by using the third-party device. The pdb file is a program data file, which may visualize the target protein substance into a three-dimensional picture, that is, may present a spatial picture of the target protein substance.

After acquiring the target protein substance provided by the third-party device, the server may identify a target protein type of the target protein substance, where the target protein type represents a type of a disease relating to the target protein substance, such as a type of cancer or a type of virus. A mapping relationship between the disease types and the corresponding variable regions in the reference protein substance is maintained on the server, where the mapping relationship indicates the corresponding variable region in the reference protein substance for each disease type.

Based on this, the server may retrieve a variable region in the reference protein substance which has a mapping relationship with the identified target protein type of the target protein substance, and then determine the retrieved variable region in the reference protein substance which corresponds to the target protein substance as a protein adjusting region in the reference protein substance.

Step S 102: Generate a predicted protein fragment at the protein adjusting region in the reference protein substance by using the protein prediction model and the protein attribute information, the protein prediction model being obtained by training based on a target protein substance; and the protein prediction model being configured to predict a protein substance for binding to the target protein substance.

Specifically, the server may call the protein prediction model obtained by training based on the target protein substance, and configured to predict the protein substance for binding to the target protein substance. The server may input the acquired protein attribute information of the reference protein substance into the protein prediction model, and then the protein prediction model may correspondingly generate the predicted protein fragment at the protein adjusting region in the reference protein substance. The predicted protein fragment is a modified protein fragment at the protein adjusting region in the reference protein substance. A protein fragment may be composed of a plurality of amino acids connected to one another.

Optionally, the server may input the whole protein attribute information of the reference protein substance into the protein prediction model so as to generate the predicted protein fragment by the protein prediction model. Alternatively, the server may input the protein attribute information at the protein adjusting region in the reference protein substance into the protein prediction model so as to generate the predicted protein fragment by the protein prediction model. The protein attribute information at the protein adjusting region in the reference protein substance may include the amino acid structure information and the amino acid torsion angle information of an amino acid at the protein adjusting region of the reference protein substance, and may also include protein sequence information of a protein fragment at the protein adjusting region of the reference protein fragment. In a specific implementation, the whole protein attribute information of the reference protein substance is inputted into the protein prediction model, or only the protein attribute information at the protein adjusting region in the reference protein substance is inputted into the protein prediction model, which may selected according to the actual application scenario.

A process of generating the predicted protein fragment at the protein adjusting region in the reference protein substance by using the protein prediction model and the protein attribute information may be as follows.

An amino acid at the protein adjusting region in the reference protein substance is determined as an adjustment amino acid. The adjustment amino acid may include a plurality of amino acids. The protein prediction model may generate predicted structure information and predicted torsion angle information of each adjustment amino acid according to the inputted protein attribute information of the reference protein substance. Each adjustment amino acid may correspond to a piece of predicted structure information and a piece of predicted torsion angle information. Each piece of predicted structure information may be structure information of any one secondary structure of four secondary structures of α-helix, β-sheet, β-turn and random coil, and each piece of predicted torsion angle information may include 3 angles including Phi angle, Psi angle and Omega angle.

Further, a specific process of predicting the predicted structure information of each adjustment amino acid by the protein prediction model and generating the predicted structure information of the adjustment amino acid by the protein prediction model may be as follows.

Since an amino acid has four types of secondary structures including α-helix, β-sheet, β-turn, and random coil, each secondary structure may be understood as a structure dimension (which may be referred to as an amino acid structure dimension) of an amino acid. Thus, the amino acid structure dimensions of an amino acid may include four amino acid structure dimensions of α-helix, β-sheet, β-turn and random coil.

Based on this, the protein prediction model may predict the sampling probability of the adjustment amino acid on each amino acid structure dimension. With regard to each adjustment amino acid, each amino acid structure dimension corresponds to a sampling probability. The sampling probability represents the sampling probability of the secondary structure of the predicted adjustment amino acid being on the corresponding amino acid structure dimension. The amino acid structure dimension with the highest sampling probability among all amino acid structure dimensions may be taken as a target structure dimension. Each adjustment amino acid corresponds to one target structure dimension.

Then, the server may sample structure parameters on the target structure dimension, to generate predicted structure information corresponding to the adjustment amino acid. The generated predicted structure information is the generated secondary structure of the adjustment amino acid on the target structure dimension. For example, if the target structure dimension is the structure dimension of α-helix, then the generated predicted structure information corresponding to the adjustment amino acid is the α-helix secondary structure of the adjustment amino acid. It is understood that a process of sampling the structure parameters is a process of generating the predicted structure information.

Further, torsion angle information of each adjustment amino acid may be predicted by the protein prediction model. The process of generating the predicted torsion angle information of the adjustment amino acid by the protein prediction model may be as follows.

The torsion angle of an amino acid may include a Phi angle, a Psi angle and an Omega angle. The Phi angle, Psi angle and Omega angle may range from 0 to 360 degrees. A sampling interval may be determined as 10 degrees. For example, [0, 10] may be a sampling interval. Therefore, each of the Phi angle, Psi angle and Omega angle corresponds to 36 (namely 360/10) sampling intervals. Each sampling interval may be understood as a torsion angle dimension (which may be referred to as an amino acid torsion angle dimension), and thus, the torsion angle of an amino acid has 36*36*36 amino acid torsion angle dimensions in total.

For example, if one sampling interval of the Phi angle is [0, 10], one sampling interval of the Psi angle is [10, 20] and one sampling interval of the Omega angle is [20, 30], then an amino acid torsion angle dimension may be Phi [0, 10] - Psi [10, 20] - Omega [20, 30], which represents that the Phi angle ranges from 0 to 10 degrees, the Psi angle ranges from 10 to 20 degrees, and the Omega angle ranges from 20 to 30 degrees.

Optionally, the sampling interval is not limited to 10 degrees, and may be other angle ranges, which is determined according to the actual application scenario, and is not limited herein. For example, the sampling interval may be 5 degrees, and [0, 5] may be a sampling interval. In this case, each of the Phi angle, the Psi angle and the Omega angle may correspond to 72 (namely 360/5) sampling intervals, and thus, the torsion angle of an amino acid has 72 * 72 * 72 amino acid torsion angle dimensions in total.

For example, if the sampling interval of the Phi angle is [0, 5], the sampling interval of the Psi angle is [5, 10] and the sampling interval of the Omega angle is [10, 15], then the amino acid torsion angle dimension may be [0, 5] - [5, 10] - [10, 15], which represents that the Phi angle ranges from 0 to 5 degrees, the Psi angle ranges from 5 to 10 degrees, and the Omega angle ranges from 10 to 15 degrees.

Based on this, the protein prediction model may predict the sampling probability of the adjustment amino acid on each amino acid torsion angle dimension. For each adjustment amino acid, each amino acid torsion angle dimension corresponds to one sampling probability. The sampling probability represents the probability of the torsion angle of the predicted adjustment amino acid being on the corresponding amino acid torsion angle dimension. The amino acid torsion angle dimension with the highest sampling probability among all the amino acid torsion angle dimensions may be taken as a target torsion angle dimension. Each adjustment amino acid corresponds to one target torsion angle dimension.

Then, the server may sample a torsion angle parameter on the target torsion angle dimensions, to generate predicted torsion angle information corresponding to the adjustment amino acid. The generated predicted torsion angle information is a generated torsion angle of the adjustment amino acid on the target torsion angle dimension. For example, torsion angle parameters may be sampled by using an intermediate angle, for example, the sampling interval may be 10 degrees, the sampling interval of the 10 degrees may be [0, 10], and then the angle obtained by sampling in the sampling interval may be 5 degrees.

For example, if an amino acid torsion angle dimension is Phi [0, 10]-Psi[10, 20]-Omega [20, 30], then the Phi angle obtained by sampling in [0, 10] may be 5 degrees, the Psi angle obtained by sampling in [10, 20] may be 15 degrees, and the Omega angle obtained by sampling in [20, 30] may be 25 degrees, and correspondingly, the predicted torsion angle information of the adjustment amino acid, which is generated finally by sampling, is a Phi angle of 5 degrees, a Psi angle of 15 degrees and an Omega angle of 25 degrees. It is understood that a process of sampling the torsion angle parameters is a process of generating the predicted torsion angle information.

By the above-mentioned process, predicted structure information and predicted torsion angle information which correspond to each adjustment amino acid are generated. On the basis of the predicted structure information and the predicted torsion angle information which correspond to each adjustment amino acid, a protein fragment at the protein adjusting region is determined, which may be referred to as a predicted protein fragment. The structure information of the predicted protein fragment is the predicted structure information of the adjustment amino acid, and the torsion angle information of the predicted protein fragment is the predicted torsion angle information of the adjustment amino acid.

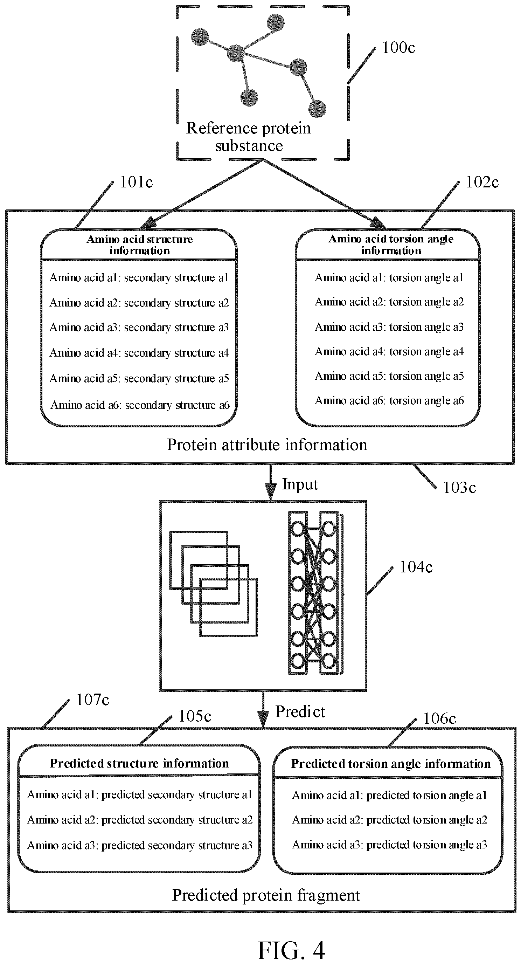

FIG. 4 is a schematic diagram of a scenario of fragment prediction according to the present disclosure. As shown in FIG. 4, it is assumed that the reference protein substance 100c includes 6 amino acids in total, which are respectively an amino acid a1, an amino acid a2, an amino acid a3, an amino acid a4, an amino acid a5 and an amino acid a6. It is assumed that amino acids at the protein adjusting region in the reference protein substance 100c include an amino acid a1, an amino acid a2 and an amino acid a3. That is, the adjustment amino acids in the reference protein substance 100c include an amino acid a1, an amino acid a2 and an amino acid a3.

The amino acid structure information for each amino acid in the reference protein substance 100c may include the actual secondary structure of each amino acid. As shown in Box 101c, the amino acid structure information includes a secondary structure a1 for the amino acid a1, a secondary structure a2 for the amino acid a2, a secondary structure a3 for the amino acid a3, a secondary structure a4 for the amino acid a4, a secondary structure a5 for the amino acid a5 and a secondary structure a6 for the amino acid a6.

The amino acid torsion angle information of each amino acid in the reference protein substance 100c may include the real torsion angle of each amino acid. As shown in Box 102c, the amino acid torsion angle information includes a torsion angle a1 of the amino acid a1, a torsion angle a2 of the amino acid a2, a torsion angle a3 of the amino acid a3, a torsion angle a4 of the amino acid a4, a torsion angle a5 of the amino acid a5 and a torsion angle a6 of the amino acid a6.

Therefore, by using the secondary structure of each amino acid in Box 101c above-mentioned and the torsion angle of each amino acid in Box 102c, protein attribute information 103c of the reference protein substance 100c may be obtained.

The server may input the protein attribute information 103c into the protein prediction model 104c, so that the protein prediction model 104c generates the predicted structure information and the predicted torsion angle information of each adjustment amino acid in the reference protein substance 100c. The predicted structure information of each adjustment amino acid may be a predicted secondary structure of each adjustment amino acid, which is predicted by the protein prediction model 104c. As shown in Box 105c, the predicted structure information of the adjustment amino acids includes a predicted secondary structure a1 of the amino acid a1, a predicted secondary structure a2 of the amino acid a2 and a predicted secondary structure a3 of the amino acid a3.

The predicted torsion angle information of each amino acid may include the predicted torsion angle of each adjustment amino acid, which is predicted by the protein prediction model. As shown in Box 106c, the predicted torsion angle information of the adjustment amino acids includes a predicted torsion angle a1 of the amino acid a1, a predicted torsion angle a2 of the amino acid a2 and a predicted torsion angle a3 of the amino acid a3.

Therefore, on the basis of the predicted structure information of each adjustment amino acid in Box 107c and the predicted torsion angle information of each adjustment amino acid in Box 106c, a predicted protein fragment 107c may be obtained.

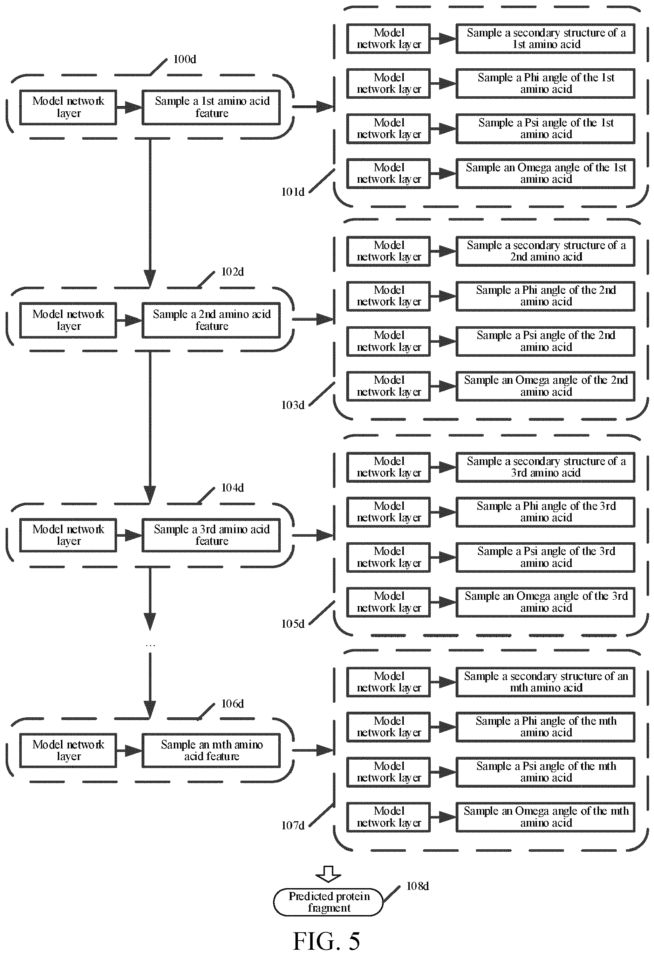

FIG. 5 is a schematic diagram of a scenario of fragment generation according to the present disclosure. As shown in FIG. 5, a 1st amino acid to an m-th amino acid may be adjustment amino acids of the reference protein substance at the protein adjusting region. In the process of generating the predicted protein fragment by the protein prediction model, the protein prediction model may sample the predicted structure information and the predicted torsion angle information of each adjustment amino acid by using a model network layer in the protein prediction model.

As shown in Box 100d, the protein prediction model may sample an amino acid feature of the 1st amino acid. As shown in Box 101d, sampling the amino acid feature of the 1st amino acid includes sampling the secondary structure of the 1st amino acid, sampling the Phi angle of the 1st amino acid, sampling the Psi angle of the 1st amino acid and sampling the Omega angle of the 1st amino acid. The secondary structure, obtained by sampling, of the 1st amino acid is the predicted structure information of the 1st amino acid, and the Phi angle, Psi angle and Omega angle, which are obtained by sampling, of the 1st amino acid are the predicted torsion angle information of the 1st amino acid.

As shown in Box 102d, the protein prediction model may sample an amino acid feature of a 2nd amino acid. As shown in Box 103d, sampling the amino acid feature of the 2nd amino acid includes sampling a secondary structure of the 2nd amino acid, sampling a Phi angle of the 2nd amino acid, sampling a Psi angle of the 2nd amino acid and sampling an Omega angle of the 2nd amino acid. The secondary structure, obtained by sampling, of the 2nd amino acid is the predicted structure information of the 2nd amino acid, and the Phi angle, Psi angle and Omega angle, which are obtained by sampling, of the 2nd amino acid are the predicted torsion angle information of the 2nd amino acid.

As shown in Box 104d, the protein prediction model may sample an amino acid feature of a 3rd amino acid. As shown in Box 105d, sampling the amino acid feature of the 3rd amino acid includes sampling a secondary structure of the 3rd amino acid, sampling a Phi angle of the 3rd amino acid, sampling a Psi angle of the 3rd amino acid and sampling an Omega angle of the 3rd amino acid. The secondary structure, obtained by sampling, of the 3rd amino acid is the predicted structure information of the 3rd amino acid, and the Phi angle, Psi angle and Omega angle, which are obtained by sampling, of the 3rd amino acid are the predicted torsion angle information of the 3rd amino acid.

By the above process, the protein prediction model may sample the amino acid feature of the adjustment amino acids one by one until the amino acid feature of the last adjustment amino acid (for example, the m-th amino acid) is obtained by sampling.

As shown in Box 106d, the protein prediction model may sample the amino acid feature of the m-th amino acid. As shown in Box 107d, sampling the amino acid feature of the m-th amino acid includes sampling a secondary structure of the m-th amino acid, sampling a Phi angle of the m-th amino acid, sampling a Psi angle of the m-th amino acid and sampling an Omega angle of the m-th amino acid. The secondary structure, obtained by sampling, of the m-th amino acid is the predicted structure information of the m-th amino acid, and the Phi angle, Psi angle and Omega angle, which are obtained by sampling, of the m-th amino acid are the predicted torsion angle information of the m-th amino acid.

Then, the protein prediction model may generate a predicted protein fragment 108d by using the amino acid features, obtained by the above-mentioned sampling, of all the adjustment amino acids (including the 1st amino acid to the m-th amino acid).

How to train the protein prediction model above-mentioned is described in detail below.

In an embodiment of the present disclosure, the protein prediction model is trained by a reinforcement learning method. Training data and prediction data of the protein prediction model are generally the same, and therefore, it is understood that the training process of the protein prediction model is equivalent to a process of continuously updating the predicted protein fragment.

In other words, each time the server acquires a target protein substance, the protein prediction model may be trained on the basis of the target protein substance and the reference protein substance, and the predicted protein fragment at the protein adjusting region in the reference protein substance is predicted in real time by using the trained protein prediction model. The predicted protein fragment is used to assist in generation of a macromolecular antibody protein for binding to the target protein substance.

In this embodiment of the present disclosure, the untrained protein prediction model may be referred to as an initial prediction model. In other words, the protein prediction model may be obtained by training the initial prediction model. The initial prediction model may be a deep neural network, such as an RNN network (recurrent neural network), an LSTM network (long short term memory network) or other neural network structures.

Similarly, since the training data is the target protein substance and the reference protein substance, the protein attribute information of the reference protein substance may be inputted into the initial prediction model, and a protein fragment at the protein adjusting region in the reference protein substance is predicted by using the initial prediction model. The protein fragment, which is predicted by the initial prediction model, at the protein adjusting region in the reference protein substance may be referred to as a predicted protein fragment sample. The principle of generating the predicted protein fragment sample by the initial prediction model is the same as the principle of generating the predicted protein fragment by the protein prediction model, but the model parameters of the initial prediction model are different from the model parameters of the protein prediction model, so that the predicted protein fragment sample generated by the initial prediction model and the predicted protein fragment generated by the protein prediction model are different.

Further, a protein fragment database is maintained in the server. The protein fragment database may include a plurality of protein fragments, each protein fragment being a short chain polypeptide. The short chain polypeptide may be composed of a plurality of amino acids, and therefore, the protein fragment database may also be referred to as a short chain polypeptide database. It is understood that, the protein fragment database may include all the short chain polypeptides which are collected before and exist in nature, and more specifically, the protein fragment database may also include protein fragments obtained by splitting proteins collected before.

After the predicted protein fragment sample generated by the initial prediction model is acquired, the server may identify a protein fragment similar to the predicted protein fragment sample in the protein fragment database, and the identified protein fragment similar to the predicted protein fragment sample may be referred to as a similar protein fragment sample. The length of the identified similar protein fragment sample is the same as the length of the predicted protein fragment sample, and the length of the predicted protein fragment sample is the same as the length of the protein fragment at the protein adjusting region in the reference protein substance. The number of amino acids may be used for measuring the length of protein fragments, and protein fragments with the same number of amino acids may be considered as protein fragments having the same length. A similar protein fragment sample matching the predicted protein fragment sample may be searched for from the protein fragment database by using a FragmentPicker tool (a fragment selecting tool).

Then, virtual synthesis is performed on the identified similar protein fragment sample and the reference protein substance to obtain synthetic substance auxiliary information sample. The virtual synthesis of the similar protein fragment sample and the reference protein substance may be performed by replacing the protein fragment at the protein adjusting region in the reference protein substance with the similar protein fragment sample, and correspondingly, the synthetic substance auxiliary information sample may be related description information (for example, description information that can describe and uniquely determine the protein substance, such as protein structure information, protein sequence information and protein torsion angle information) of the protein substance obtained by synthesizing the similar protein fragment sample and the reference protein substance, that is, related description information of a new protein substance obtained by replacing the protein fragment at the protein adjusting region in the reference protein substance with the similar protein fragment sample.

It is understood that, the synthetic substance auxiliary information sample is used for assisting in generation of an antibody protein substance sample for binding to the target protein substance. The antibody protein substance sample is a protein substance obtained by replacing the protein fragment at the protein adjusting region in the reference protein substance with a similar protein fragment sample, and is a macromolecular antibody protein which is generated on the basis of the identified similar protein fragment sample and is configured to bind to the target protein substance.

Then, the server may further acquire a binding strength between the antibody protein substance sample and the target protein substance, and the binding strength may be referred to as binding strength sample. As implied by the name, the binding strength sample is the strength of binding caused by reaction between the sample antibody protein fragment substance and the target protein substance.

A higher binding strength between the antibody protein substance and the target protein substance indicates better therapeutic effect of the antibody protein substance on the disease relating to the target protein substance. Therefore, the binding strength sample between the antibody protein substance sample and the target protein substance obtained by the server may be used for providing an excitation parameter or a penalty parameter to the initial prediction model to modify the model parameters of the initial prediction model, as described below.

The server may perform model training on the initial prediction model for a plurality of times on the basis of the reference protein substance and the target protein substance. Each time when performing the model training on the initial prediction model, the initial prediction model generates a predicted protein fragment sample, and then a binding strength sample is obtained. Therefore, correcting the model parameters of the initial prediction model on the basis of the binding strength samples may include correcting the model parameters of the initial prediction model on the basis of difference between the binding strength samples obtained in adjacent rounds of model training of the initial prediction model.

Specifically, when the binding strength sample obtained by a current round of training of the initial prediction model is greater than the binding strength sample obtained by the previous round of training of the initial prediction model, it indicates that the accuracy of the predicted protein fragment sample obtained by prediction of the current round of training is higher than the accuracy of the predicted protein fragment sample obtained by prediction of the previous round of training, so that a reward parameter may be given to the initial prediction model in the next training process to encourage the initial prediction model to correct the model parameters in a good prediction direction (i.e., the prediction direction of the current time) on the basis of the reward parameter.

Conversely, when the binding strength sample obtained by the current round of training of the initial prediction model is less than the binding strength sample obtained by the previous round of training of the initial prediction model, it indicates that the accuracy of the predicted protein fragment sample obtained by prediction of the current round of training is lower than the accuracy of the predicted protein fragment sample obtained by prediction of the previous round of training, so that a penalty parameter may be given to the initial prediction model in the next training process, to control the initial prediction model to correct the model parameters in the good prediction direction (i.e., the previous prediction direction) instead of the poor prediction direction (i.e., the current prediction direction) according to the penalty parameter.

For example, in the n-th training process of the initial prediction model, the server may synthesize an antibody protein substance sample kₙ by using the similar protein fragment sample matching the predicted protein fragment sample generated by the initial prediction model. The binding strength sample between the antibody protein substance sample kₙ and the target protein substance is the binding strength sample qₙ. n may be a positive integer less than or equal to the total number of training times for the initial prediction model.

In the (n-1)-th training process of the initial prediction model, the server may synthesize an antibody protein substance sample kₙ₋₁ by using a similar protein fragment sample matching the predicted protein fragment sample generated by the initial prediction model, and the binding strength sample between the antibody protein substance sample kₙ₋₁ and the target protein substance may be binding strength sample qₙ₋₁.

Optionally, in the n-th training process of the initial prediction model, the server may search the protein fragment database for a plurality of similar protein fragment samples matching the predicted protein fragment sample, where the plurality of similar protein fragment samples may be N protein fragments that are most similar to the predicted protein fragment sample in the protein fragment database, and a specific value of N may be determined according to an actual application scenario, which is not limited herein.

Each similar protein fragment sample corresponds to an antibody protein substance sample kₙ. Therefore, a plurality (for example, N) of antibody protein substance samples kₙ exist, and each of the antibody protein substance samples kₙ has a binding strength (which may be referred to as a target binding strength) with the target protein substance, and therefore, an average value (which may be referred to as an average strength) of the target binding strengths between the antibody protein substance samples kₙ and the target protein substance may be used as the binding strength sample qₙ in the n-th training process. Similarly, the binding strength sample qₙ₋₁ in the (n-1)-th training process may be calculated by the method for calculating the binding strength sample qₙ.

Then, the server may correct the model parameter of the initial prediction model on the basis of the binding strength sample qₙ and the binding strength sample qₙ₋₁. For example, the server may acquire a squared difference between the binding strength sample qₙ and the binding strength sample qₙ₋₁, where the squared difference may be the value of the square of the binding strength sample qₙ minus the square of the binding strength sample qₙ₋₁.

The penalty parameter and the reward parameter may be collectively referred to as an excitation parameter, and the server may give the squared difference between the binding strength sample qₙ and the binding strength sample qₙ₋₁ to the initial prediction model as an excitation parameter, so that the initial prediction model corrects its model parameters on the basis of the excitation parameter.

It is understood that, if the binding strength sample qₙ is greater than the binding strength sample qₙ₋₁, the value of the squared difference between the binding strength sample qₙ and the binding strength sample qₙ₋₁ is a positive number, and at this time, the squared difference is used as a reward parameter to the initial prediction model, so that the initial prediction model corrects its model parameters by using the reward parameter. If the binding strength sample qₙ is less than the binding strength sample qₙ₋₁, the value of the squared difference between the binding strength sample qₙ and the binding strength sample qₙ₋₁ is a negative number, and at this time, the squared difference is used as a penalty parameter to the initial prediction model, so that the initial prediction model corrects its model parameters by using the penalty parameter.

By the above-mentioned process, the initial prediction model may be continuously trained by using the target protein substance and the reference protein substance, and when the initial prediction model converges or the time of training of the initial prediction model reaches a certain threshold, the initial prediction model trained at the moment may be used as the above-mentioned protein prediction model.

It is understood that, since each training of the initial prediction model corrects the model parameters of the initial prediction model, the predicted protein fragment samples obtained by prediction by the initial prediction model in each training process are usually different, and correspondingly, the binding strength sample obtained in each training process is also usually different, and the model parameters of the initial prediction model may be continuously corrected on the basis of the different binding strength samples obtained in each training process. This is equivalent to that sample data (which may be the binding strength sample in each training process) used for training the initial prediction model is generated by the initial prediction model itself, so that it is not necessary to prepare a large amount of sample data.

The binding strength sample between the antibody protein substance sample and the target protein substance may be obtained by performing protein-docking (an algorithm for calculating the interaction relationship between molecular substances) calculation on the antibody protein substance sample and the target protein substance.

From above, by the method provided by this embodiment of the present disclosure, the initial prediction model may be given the excitation parameter on the basis of the binding strength sample between the target protein substance and the antibody protein substance sample synthesized by using the synthetic substance auxiliary information sample, so that the initial prediction model performs reinforcement learning by using the excitation parameter, to obtain the final protein prediction model. By the protein prediction model, the protein substance for binding to the target protein substance can be automatically predicted on the basis of the AI technology.

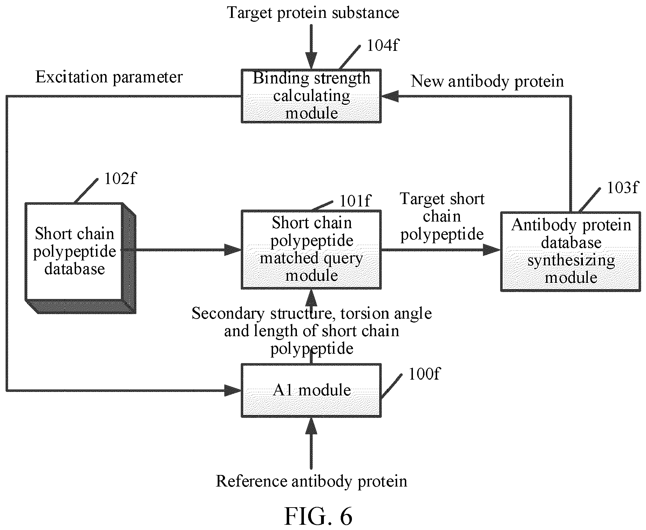

Referring to FIG. 6, FIG. 6 is a schematic diagram of a scenario of model training according to the present disclosure. As shown in FIG. 6, an AI module 100f refers to the initial prediction model, and in the present disclosure, besides the AI module, a short chain polypeptide matching module 101f, an antibody protein data synthesizing module 103f and a binding strength calculating module 104f are further included.

The server may input the protein attribute information of the reference antibody protein into the AI module, to predict a secondary structure (namely the sample predicted structure information) and a torsion angle (namely the sample predicted torsion angle information) of the amino acid at the protein adjusting region by the AI module.

On the basis of the length of the original protein fragment (namely the length of short chain polypeptide) at the protein adjusting region and the secondary structure and torsion angle of the amino acid at the protein adjusting region predicted by the AI module, a similar protein fragment sample may be identified from a short chain polypeptide database 102f. The similar protein fragment sample is a target short chain polypeptide here.

The server may perform synthesis on the target short chain polypeptide and the reference antibody protein by using the antibody protein data synthesizing module 103f to obtain a new antibody protein, where the new antibody protein is the antibody protein substance sample above-mentioned. Then, the server may calculate the binding strength between the new antibody protein and the target protein substance by using the binding strength calculating module 104f, and generate an excitation parameter for the AI module by using the binding strength. The server may give the excitation parameter to the AI module to correct the model parameters of the AI module.

Through the above-mentioned process, the AI module 100f may be trained continuously and cyclically on the basis of the reference antibody protein and the target protein substance, and the trained AI module 100f is the above-mentioned protein prediction model.

Step S103: Identify a similar protein fragment matching the predicted protein fragment from the protein fragment database.

Specifically, the server may identify a protein fragment similar to the predicted protein fragment predicted by the protein prediction model from the protein fragment database. The protein fragment similar to the predicted protein fragment identified from the protein fragment database may be referred to as a similar protein fragment to the predicted protein fragment. The length of the similar protein fragment is the same as the length of the predicted protein fragment. The similar protein fragment matching the predicted protein fragment may be identified from the protein fragment database by using a FragmentPicker tool (fragment selecting tool).

In this embodiment of the present disclosure, the data size of the protein fragment database for identifying the similar protein fragment may be large. The predicted protein fragments obtained by using the protein prediction model provide the direction and basis of protein retrieval. Therefore, even if the number of protein fragments in the protein fragment database is extremely large, similar protein fragments having similar secondary structures and similar torsion angles to the secondary structure (namely the predicted structure information above-mentioned) and the torsion angle (namely the predicted torsion angle information above-mentioned) of the predicted protein fragment can be quickly retrieved from the protein fragment database.

The similar protein fragment is a protein fragment which is identified from the protein fragment database and is the most similar to the secondary structure and torsion angle of the predicted protein fragment. When identifying the similar protein fragment matching the predicted protein fragment from the protein fragment database, weights may be set for the predicted structure information and the predicted torsion angle information. The weight set for the predicted structure information may be referred to as a structure weight, and the weight set for the predicted torsion angle information may be referred to as a torsion angle weight. Therefore, the protein fragment having the highest overall similarity to the predicted structure information and the predicted torsion angle information may be identified from the protein fragment database according to the structure weight and the torsion angle weight, as the similar protein fragment to the predicted protein fragment.

Step S104: Perform virtual synthesis on the similar protein fragment and the reference protein substance to obtain synthetic substance auxiliary information, the synthetic substance auxiliary information being used for assisting in generation of an antibody protein substance for binding to the target protein substance.

Specifically, the server may perform virtual synthesis on the identified similar protein fragment and the reference protein substance to obtain the synthetic substance auxiliary information. The virtual synthesis on the similar protein fragment and the reference protein substance may be performed by replacing the protein fragment at the protein adjusting region in the reference protein substance with the similar protein fragment. The virtual synthesis on the similar protein fragment and the reference protein substance refers to simulating a synthesis process on the similar protein fragment and the reference protein substance by a computing device instead of actually synthesizing an antibody protein substance by using a real similar protein fragment and the reference protein substance.

The synthetic substance auxiliary information may be related description information of a new protein substance obtained by performing the synthesis on the similar protein substance and the reference protein substance (for example, description information such as protein structure information, protein sequence information and protein torsion angle information that can be used for describing and uniquely determining the protein substance). The synthetic substance auxiliary information is the related description information of the new protein substance obtained by replacing the protein fragment at the protein adjusting region in the reference protein substance with the similar protein fragment. The similar protein fragment and the reference protein substance may be virtually combined by a PyRosetta tool, where PyRosetta is a Rosetta (a table logic data tool based on a rough set theory framework) interaction suite based on Python (a computer programming language).

It is understood that, the synthetic substance auxiliary information is used for assisting in generation of an antibody protein substance for binding to the target protein substance, the antibody protein substance is a protein substance obtained by replacing the protein fragment at the protein adjusting region in the reference protein substance with the similar protein fragment. The antibody protein substance is a macromolecular antibody protein which is generated on the basis of the similar protein fragment and is configured to bind to the target protein substance. The antibody protein substance is a finally predicted medicine for treating the disease relating to the target protein substance, and the antibody protein substance may bind to the target protein substance to realize treatment of the disease relating to the target protein substance.

For example, the server may cut off a protein fragment at the protein adjusting region in the reference protein substance to obtain a cut reference protein substance. The cut reference protein substance is a protein substance after the protein fragment at the protein adjusting region of the reference protein substance is removed. Then, the server may perform virtual synthesis on the cut reference protein substance and the similar protein fragment to obtain synthetic substance auxiliary information. Virtual synthesis on the cut reference protein substance and the similar protein fragment may be performed by splicing the similar protein fragment to the cut reference protein substance at the protein adjusting region.

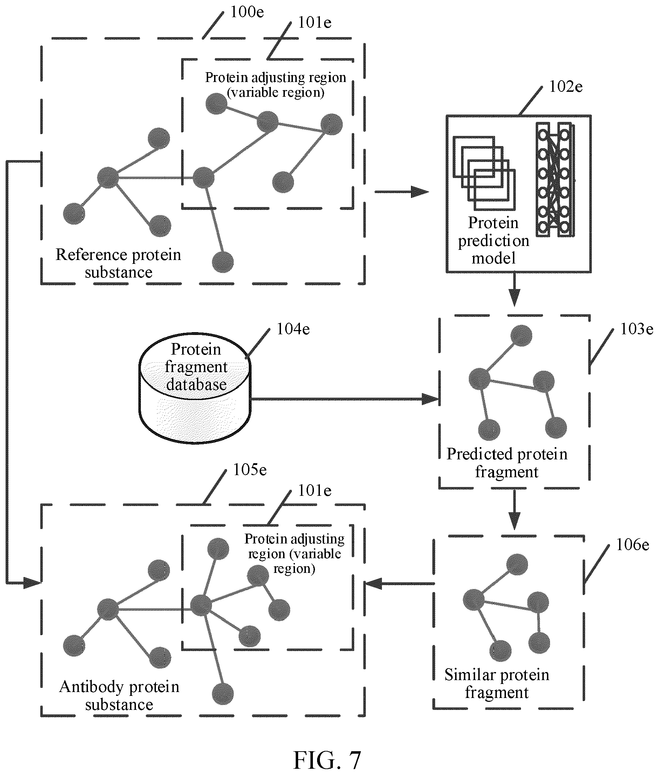

FIG. 7 is a schematic diagram of a scenario of synthesis of an antibody protein substance according to the present disclosure. As shown in FIG. 7, a reference protein substance 100e may include a variable region 101e, and the variable region 101e is a protein adjusting region in the reference protein substance 100e.

The server may input the protein attribute information of the reference protein substance into a protein prediction model 102e, and correspondingly, the protein prediction model 102e may generate a predicted protein fragment 103e for a protein fragment at the protein adjusting region 101e. The fragment length of the predicted protein fragment 103e is the same as the fragment length of an original protein fragment at the protein adjusting region 101e in the reference protein substance 100e.

Then, the server may identified a similar protein fragment 106e matching the predicted protein fragment 103e from a protein fragment database 104e. Then, the server may perform virtual synthesis on the reference protein substance 100e and the similar protein fragment 106e to obtain synthetic substance auxiliary information. The synthetic substance auxiliary information may be related description information of antibody protein substances to be synthesized, and by the synthetic substance auxiliary information, a new antibody substance (namely the antibody protein substance 105e) obtained by performing synthesis on the reference protein substance 100e and the similar protein fragment 106e may be determined. As shown in FIG. 7, the antibody protein substance 105e is a protein substance obtained by replacing an original protein fragment at the protein adjusting region 101e in the reference protein substance 100e with the similar protein fragment.

Further, the server may generate a visual program file by using the synthetic substance auxiliary information, and the file format of the visual program file may be pdb (program data file) file format. By using the visual program file, the device can visualize the antibody protein substance generated according to the synthetic substance auxiliary information. For example, by using the visual program file, a complete space picture of the antibody protein substance may be presented by the device, where the space picture may be a three-dimensional structure of the antibody protein substance.

Since the target protein substance may be provided by a third-party device for a pharmaceutical factory, the server may transmit the generated visual program file of the synthetic substance auxiliary information to the third-party device. After the third-party device acquires the visual program file of the synthetic substance auxiliary information, the visual program file may be outputted in a device page, so that related researchers may research, develop, generate and improve the antibody protein substance according to the three-dimensional picture of the presented antibody protein substance. The target protein substance may be transmitted to the server in an on-line manner by the third-party device by using related data transmitting technologies such as a cloud technology. Similarly, the server may predict and generate the synthetic substance auxiliary information in an on-line manner by using related technologies such as a cloud technology, and transmit the visual program file to the third-party device in an on-line manner by using related data transmitting technologies such as a cloud technology.

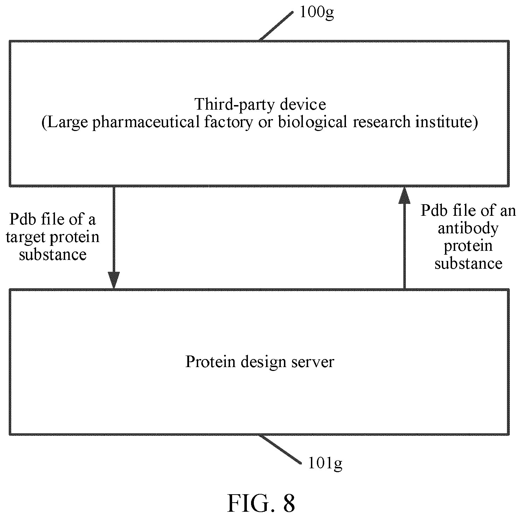

FIG. 8 is a schematic diagram of a scenario of data interaction according to the present disclosure. The third-party device 100g may be a device for a large pharmaceutical factory or biological research institute. The third-party device 100g may provide the pdb file of the target protein substance to a protein design server 101g (namely the server that is the execution subject in the present disclosure). Further, the protein design server 101g may generate the synthetic substance auxiliary information on the basis of the pdb file, provided by the third-party device, of the target protein substance, and may generate a pdb file for the antibody protein substance, that is, the visual program file, on the basis of the synthetic substance auxiliary information.

The protein design server 101g may transmit the generated pdb file of the antibody protein substance to the third-party device 100g, so that the third-party device 100g may download the pdb file of the antibody protein substance with payment, and output the pdb file of the antibody protein substance in the device page after the download with payment. In this way, the three-dimensional space picture of the antibody protein substance is presented, and is provided for relevant researchers to research, modify or develop the antibody protein substance.

In the present disclosure, the protein attribute information of the reference protein substance is acquired. The reference protein substance includes the protein adjusting region. A predicted protein fragment at the protein adjusting region in the reference protein substance is generated according to the protein attribute information by using a protein prediction model. The protein prediction model is obtained by training on the basis of a target protein substance, and is configured to predict the protein substance for binding to the target protein substance. The similar protein fragment matching the predicted protein fragment is identified from the protein fragment database. Virtual synthesis is performed on the similar protein fragment and the reference protein substance to obtain synthetic substance auxiliary information. The synthetic substance auxiliary information is used for assisting in generation of an antibody protein substance for binding to the target protein substance. Therefore, according to the method provided by the present disclosure, the similar protein fragment used to modify the protein fragment at the protein adjusting region in the reference protein substance is rapidly determined on the basis of the predicted protein fragment predicted by the protein prediction model, and the antibody protein substance configured to bind to the target protein substance can be rapidly generated on the basis of the similar protein fragment, thereby improving the efficiency of acquiring the antibody protein substance.

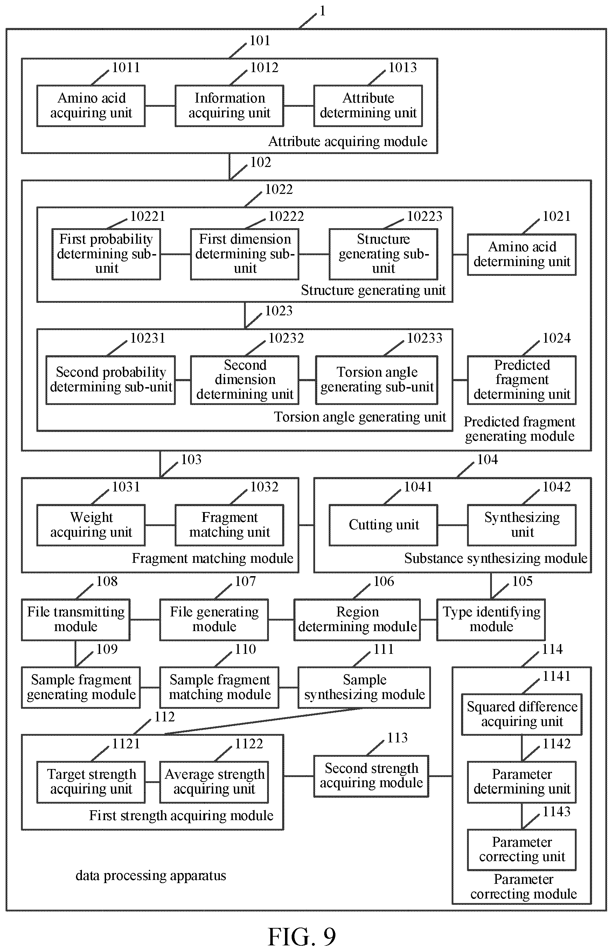

FIG. 9 is a schematic structural diagram of a data processing apparatus according to the present disclosure. As shown in FIG. 9, the data processing apparatus 1 may include: an attribute acquiring module 101, a predicted fragment generating module 102, a fragment matching module 103 and a substance synthesizing module 104.

The attribute acquiring module 101 is configured to acquire protein attribute information of a reference protein substance. The reference protein substance includes a protein adjusting region.

The predicted fragment generating module 102 is configured to generate, by using a protein prediction model, a predicted protein fragment at the protein adjusting region in the reference protein substance according to the protein attribute information, the protein prediction model being obtained by training based on a target protein substance, and the protein prediction model being configured to predict a protein substance for binding to a target protein substance.

The fragment matching module 103 is configured to identify a similar protein fragment matching the predicted protein fragment from a protein fragment database.

The substance synthesizing module 104 is configured to perform virtual synthesis on the similar protein fragment and the reference protein substance to obtain synthetic substance auxiliary information, where the synthetic substance auxiliary information being used for assisting in generation of an antibody protein substance for binding to the target protein substance.

For a specific functional implementation of the attribute acquiring module 101, the predicted fragment generating module 102, the fragment matching module 103 and the substance synthesizing module 104, reference may be made to steps S101 to S104 in the embodiment corresponding to FIG. 3, and details are not described herein again.

The attribute acquiring module 101 may include: an amino acid acquiring unit 1011, an information acquiring unit 1012 and an attribute determining unit 1013.

The amino acid acquiring unit 1011 is configured to acquire at least two amino acids included in the reference protein substance.

The information acquiring unit 1012 is configured to acquire the amino acid structure information and the amino acid torsion angle information of each amino acid in the at least two amino acids.

The attribute determining unit 1013 is configured to determine the amino acid structure information and the amino acid torsion angle information of each amino acid as the protein attribute information of the reference protein substance.

For a specific functional implementation of the amino acid acquiring unit 1011, the information acquiring unit 1012 and the attribute determining unit 1013, reference may be made to step S101 in the embodiment corresponding to FIG. 3, and details are not described herein again.

The predicted fragment generating module 102 may include: an amino acid determining unit 1021, a structure generating unit 1022, a torsion angle generating unit 1023 and a predicted fragment determining unit 1024.

The amino acid determining unit 1021 is configured to determine an amino acid at the protein adjusting region in the reference protein substance as an adjustment amino acid.

The structure generating unit 1022 is configured to generate predicted structure information corresponding to the adjustment amino acid by using the protein prediction model.

The torsion angle generating unit 1023 is configured to generate predicted torsion angle information corresponding to the adjustment amino acid by using the protein prediction model.

The predicted fragment determining unit 1024 is configured to determine the predicted protein fragment according to the predicted structure information and predicted torsion angle information corresponding to the adjustment amino acid.

For a specific functional implementation of the amino acid determining unit 1021, the structure generating unit 1022, the torsion angle generating unit 1023 and the predicted fragment determining unit 1024, reference may be made to step S102 in the embodiment corresponding to FIG. 3, and details are not described herein again.

The structure generating unit 1022 may include: a first probability determining sub-unit 10221, a first dimension determining sub-unit 10222 and a structure generating sub-unit 10223.

The first probability determining sub-unit 10221 is configured to determine, by using the protein prediction model, a sampling probability of the adjustment amino acid on each amino acid structure dimension of at least two amino acid structure dimensions.

The first dimension determining sub-unit 10222 is configured to determine an amino acid structure dimension with the highest sampling probability among the at least two amino acid structure dimensions as a target structure dimension.

The structure generating sub-unit 10223 is configured to sample structure parameters on the target structure dimension to generate predicted structure information corresponding to the adjustment amino acid.

For a specific functional implementation of the first probability determining sub-unit 10221, the first dimension determining sub-unit 10222 and the structure generating sub-unit 10223, reference may be made to step S102 in the embodiment corresponding to FIG. 3, and details are not described herein again.

The torsion angle generating unit 1023 may include: a second probability determining sub-unit 10231, a second dimension determining unit 10232 and a torsion angle generating sub-unit 10233.

The second probability determining sub-unit 10231 is configured to determine, by using the protein prediction model, a sampling probability of the adjustment amino acid on each amino acid torsion angle dimension in at least two amino acid torsion angle dimensions.

The second dimension determining unit 10232 is configured to determine an amino acid torsion angle dimension with the highest sampling probability among the at least two amino acid torsion angle dimensions as a target torsion angle dimension.

The torsion angle generating sub-unit 10233 is configured to sample a torsion angle parameter on the target torsion angle dimension and generate predicted torsion angle information corresponding to the adjustment amino acid.

For a specific functional implementation of the second probability determining sub-unit 10231, the second dimension determining unit 10232 and the torsion angle generating sub-unit 10233, reference may be made to step S102 in the embodiment corresponding to FIG. 3, and details are not described herein again.

The fragment matching module 103 may include: a weight acquiring unit 1031 and a fragment matching unit 1032.

The weight acquiring unit 1031 is configured to acquire a structure weight for the predicted structure information, and acquire a torsion angle weight for the predicted torsion angle information.

The fragment matching unit 1032 is configured to identify the similar protein fragment matching the predicted protein fragment from the protein fragment database according to the structure weight, the torsion angle weight, the predicted structure information and the predicted torsion angle information.

For a specific functional implementation of the weight acquiring unit 1031 and the fragment matching unit 1032, reference may be made to step S103 in the embodiment corresponding to FIG. 3, and details are not described herein again.

The substance synthesizing module 104 may include: a cutting unit 1041 and a synthesizing unit 1042.

The cutting unit 1041 is configured to cut off a protein fragment at the protein adjusting region in the reference protein substance to obtain a cut reference protein substance.

The synthesizing unit 1042 is configured to perform virtual synthesis on the cut reference protein substance and the similar protein fragment to obtain the synthetic substance auxiliary information.

For a specific functional implementation of the cutting unit 1041 and the synthesizing unit 1042, reference may be made to step S104 in the embodiment corresponding to FIG. 3, and details are not described herein again.

The data processing apparatus 1 may further include: a type identifying module 105 and a region determining module 106.

The type identifying module 105 is configured to identify a target protein type of the target protein substance.

The region determining module 106 is configured to determine the protein adjusting region in the reference protein substance according to the target protein type.

For a specific functional implementation of the type identifying module 105 and the region determining module 106, reference may be made to step S101 in the embodiment corresponding to FIG. 3, and details are not described herein again.

The target protein substance may be provided by a third-party device.

The data processing apparatus 1 may further include: a file generating module 107 and a file transmitting module 108.

The file generating module 107 is configured to generate a visual program file of the synthetic substance auxiliary information.

The file transmitting module 108 is configured to transmit the visual program file to the third-party device, so that the third-party device outputs the visual program file.

For a specific functional implementation of the file generating module 107 and the file transmitting module 108, reference may be made to step S104 in the embodiment corresponding to FIG. 3, and details are not described herein again.

The data processing apparatus 1 may further include: a fragment sample generating module 109, a fragment sample matching module 110, a sample synthesizing module 111, a first strength acquiring module 112, a second strength acquiring module 113 and a parameter correcting module 114.

The fragment sample generating module 109 is configured to input the protein attribute information into an initial prediction model, and generate a predicted protein fragment sample at the protein adjusting region in an n-th training process of the initial prediction model, where n is an integer greater than 1.

The fragment sample matching module 110 is configured to identify a similar protein fragment sample matching the predicted protein fragment sample from the protein fragment database.

The sample synthesizing module 111 is configured to perform virtual synthesis on the reference protein substance and the similar protein fragment sample to obtain the synthetic substance auxiliary information sample. The synthetic substance auxiliary information sample is used for assisting in generation of an antibody protein substance sample kₙ for binding to the target protein substance.

The first strength acquiring module 112 is configured to acquire a binding strength sample qₙ between the antibody protein substance sample kₙ and the target protein substance.

The second strength acquiring module 113 is configured to acquire a binding strength sample qₙ₋₁ between the antibody protein substance sample kₙ₋₁ for the target protein substance and the target protein substance in a (n-1)-th training process of the initial prediction model.

The parameter correcting module 114 is configured to correct a model parameter of the initial prediction model according to the binding strength sample qₙ and the binding strength sample qₙ₋₁ to obtain the protein prediction model.

For a specific functional implementation of the fragment sample generating module 109, the fragment sample matching module 110, the sample synthesizing module 111, the first strength acquiring module 112, the second strength acquiring module 113 and the parameter correcting module 114, reference may be made to step S102 in the embodiment corresponding to FIG. 3, and details are not described herein again.

A quantity of the similar protein fragment sample is at least two. The at least two similar protein fragment samples respectively correspond to at least two antibody protein substance samples kₙ.

The first strength acquiring module 112 includes: a target strength acquiring unit 1121 and an average strength acquiring unit 1122.

The target strength acquiring unit 1121 is configured to, for each of the at least two antibody protein substance samples kₙ, a target binding strength between the antibody protein substance sample kₙ and the target protein substance.

The average strength acquiring unit 1122 is configured to determine an average strength of target binding strengths corresponding to the at least two antibody protein substance samples kₙ as the binding strength sample qₙ.

For a specific functional implementation of the target strength acquiring unit 1121 and the average strength acquiring unit 1122, reference may be made to step S102 in the embodiment corresponding to FIG. 3, and details are not described herein again.

The parameter correcting module 114 includes: a squared difference acquiring unit 1141, a parameter determining unit 1142 and a parameter correcting unit 1143.

The squared difference acquiring unit 1141 is configured to acquire a squared difference between the binding strength sample qₙ and the binding strength sample qₙ₋₁.

The parameter determining unit 1142 is configured to determine an excitation parameter for the initial prediction model according to the squared difference.

The parameter correcting unit 1143 is configured to correct the model parameters of the initial prediction model according to the excitation parameter to obtain the protein prediction model.

For a specific functional implementation of the squared difference acquiring unit 1141, the parameter determining unit 1142 and the parameter correcting unit 1143, reference may be made to step S102 in the embodiment corresponding to FIG. 3, and details are not described herein again.

In the present disclosure, the protein attribute information of the reference protein substance is acquired. The reference protein substance includes the protein adjusting region. The predicted protein fragment at the protein adjusting region of the reference protein substance is generated according to the protein attribute information by using the protein prediction model. The protein prediction model is obtained by training on the basis of a target protein substance, and is configured to predict a protein substance for binding to the target protein substance. The similar protein fragment matching the predicted protein fragment is identified from the protein fragment database. Virtual synthesis is performed on the similar protein fragment and the reference protein substance to obtain synthetic substance auxiliary information. The synthetic substance auxiliary information is used for assisting in generation of an antibody protein substance for binding to the target protein substance. Therefore, by the apparatus provided by the present disclosure, the similar protein fragment used to modify the protein fragment at the protein adjusting region in the reference protein substance may be rapidly determined on the basis of the predicted protein fragment predicted by the protein prediction model, and the antibody protein substance for binding to the target protein substance can be rapidly generated on the basis of the similar protein fragment, thereby improving the efficiency of acquiring the antibody protein substance.

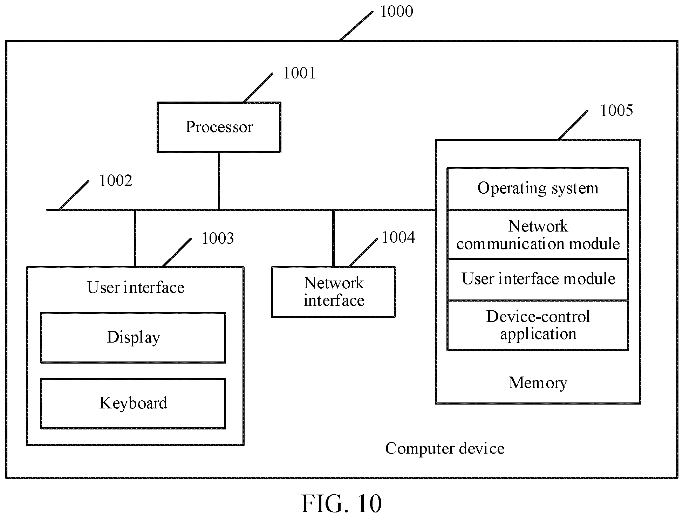

FIG. 10 is a schematic structural diagram of a computer device according to the present disclosure. As shown in FIG. 10, a computer device 1000 may include: a processor 1001, a network interface 1004, and a memory 1005. In addition, the computer device 1000 may further include: a user interface 1003 and at least one communication bus 1002. The communication bus 1002 is configured to implement connection and communication between the components. The user interface 1003 may include a display, a keyboard, and optionally, the user interface 1003 may further include a standard wired interface and a standard wireless interface. Optionally, the network interface 1004 may include a standard wired interface and a standard wireless interface (such as a Wi-Fi interface). The memory 1005 may be a high-speed RAM memory, or may be a non-volatile memory, for example, at least one magnetic disk memory. Optionally, the memory 1005 may be further at least one storage apparatus away from the foregoing processor 1001. As shown in FIG. 10, the memory 1005, which is used as a computer storage medium, may include an operating system, a network communication module, a user interface module, and a device control application program.

In the computer device 1000 shown in FIG 10, the network interface 1004 may provide a network communication function; the user interface 1003 is mainly configured to provide an input interface for a user; and the processor 1001 may be configured to invoke the device-control application stored in the memory 1005, to implement the description of the data processing method according to the corresponding embodiments in the foregoing FIG. 3. It is to be understood that the computer device 1000 described in the present disclosure can implement the descriptions of the data processing apparatus 1 in the foregoing embodiment corresponding to FIG. 9. Details are not described herein again. In addition, the description of beneficial effects of the same method is not repeated herein again.

In addition, the embodiments of the present disclosure further provide a computer-readable storage medium. The computer-readable storage medium stores a computer program executed by the data processing apparatus 1 mentioned above, and the computer program includes program instructions. When executing the program instructions, the processor can perform the descriptions of data processing method in the embodiment corresponding to FIG. 3. Therefore, details are not described herein again. In addition, the description of beneficial effects of the same method is not described herein again. For technical details that are not disclosed in the computer storage medium embodiments of the present disclosure, one may refer to the descriptions of the method embodiments of the present disclosure.

A person of ordinary skill in the art may understand that all or some of the processes of the methods in the embodiments may be implemented by a computer program instructing relevant hardware. The program may be stored in a computer-readable storage medium. When the program is executed, the procedures of the foregoing method embodiments are performed. The foregoing storage medium may include a magnetic disc, an optical disc, a read-only memory (ROM), a random access memory (RAM), or the like.

What is disclosed above is merely exemplary embodiments of the present disclosure, and certainly is not intended to limit the scope of the claims of the present disclosure. Therefore, equivalent variations made in accordance with the claims of the present disclosure shall fall within the scope of the present disclosure.

## Claims

1. A data processing method, performed by a computer device, the method comprising:
acquiring protein attribute information of a reference protein substance, the reference protein substance comprising a protein adjusting region;
generating, by using a protein prediction model, a predicted protein fragment at the protein adjusting region in the reference protein substance according to the protein attribute information, the protein prediction model being obtained by training based on a target protein substance, and the protein prediction model being configured to predict a protein substance for binding to a target protein substance;
identifying a similar protein fragment matching the predicted protein fragment from a protein fragment database; and
performing virtual synthesis on the similar protein fragment and the reference protein substance to obtain synthetic substance auxiliary information, the synthetic substance auxiliary information being used for assisting in generation of an antibody protein substance for binding to the target protein substance.

2. The method according to claim 1, wherein the acquiring the protein attribute information of the reference protein substance comprises:
acquiring at least two amino acids comprised in the reference protein substance;
acquiring amino acid structure information and amino acid torsion angle information of each amino acid in the at least two amino acids; and
determining the amino acid structure information and the amino acid torsion angle information of each amino acid as the protein attribute information of the reference protein substance.

3. The method according to claim 2, wherein the generating, by the protein prediction model, the predicted protein fragment at the protein adjusting region in the reference protein substance according to the protein attribute information comprises:
determining an amino acid at the protein adjusting region in the reference protein substance as an adjustment amino acid;
generating, by using the protein prediction model, predicted structure information corresponding to the adjustment amino acid;
generating, by using the protein prediction model, predicted torsion angle information corresponding to the adjustment amino acid; and
determining the predicted protein fragment according to the predicted structure information and the predicted torsion angle information corresponding to the adjustment amino acid.

4. The method according to claim 3, wherein the generating, by using the protein prediction model, the predicted structure information corresponding to the adjustment amino acid comprises:
determining, by using the protein prediction model, a sampling probability of the adjustment amino acid on each amino acid structure dimension of at least two amino acid structure dimensions;
determining an amino acid structure dimension with the highest sampling probability among the at least two amino acid structure dimensions as a target structure dimension; and
sampling structure parameters on the target structure dimension to generate predicted structure information corresponding to the adjustment amino acid.

5. The method according to claim 3, wherein the generating, by using the protein prediction model, the predicted torsion angle information corresponding to the adjustment amino acid comprises:
determining, by using the protein prediction model, a sampling probability of the adjustment amino acid in each amino acid torsion angle dimension of at least two amino acid torsion angle dimensions;
determining an amino acid torsion angle dimension with the highest sampling probability among the at least two amino acid torsion angle dimensions as a target torsion angle dimension; and
sampling a torsion angle parameter on the target torsion angle dimension to generate predicted torsion angle information corresponding to the adjustment amino acid.

6. The method according to claim 3, wherein the identifying the similar protein fragment matching the predicted protein fragment from the protein fragment database comprises:
acquiring a structure weight for the predicted structure information, and acquiring a torsion angle weight for the predicted torsion angle information; and
identifying the similar protein fragment matching the predicted protein fragment from the protein fragment database according to the structure weight, the torsion angle weight, the predicted structure information and the predicted torsion angle information.

7. The method according to claim 1, wherein the performing virtual synthesis on the similar protein fragment and the reference protein substance to obtain the synthetic substance auxiliary information comprises:
cutting off a protein fragment at the protein adjusting region in the reference protein substance to obtain a cut reference protein substance; and
performing virtual synthesis on the cut reference protein substance and the similar protein fragment to obtain the synthetic substance auxiliary information.

8. The method according to claim 1, further comprising:
identifying a target protein type of the target protein substance; and
determining the protein adjusting region in the reference protein substance according to the target protein type.

9. The method according to claim 1, wherein the target protein substance is provided by a third-party device; and
the method further comprises:
generating a visual program file of the synthetic substance auxiliary information; and
transmitting the visual program file to the third-party device so that the third-party device outputs the visual program file.

10. The method according to claim 1, wherein the protein prediction model is obtained by training in the following manner:
inputting the protein attribute information into an initial prediction model, and generating a predicted protein fragment sample at the protein adjusting region in an n-th training process of the initial prediction model, wherein n is an integer greater than 1;
identifying a similar protein fragment sample matching the predicted protein fragment sample from the protein fragment database;
performing virtual synthesis on the reference protein substance and the similar protein fragment sample to obtain synthetic substance auxiliary information sample; the synthetic substance auxiliary information sample being used for assisting in generation of an antibody protein substance sample kₙ for binding to the target protein substance;
acquiring a binding strength sample qₙ between the antibody protein substance sample kₙ and the target protein substance;
acquiring a binding strength sample qₙ₋₁ between an antibody protein substance sample kₙ₋₁ for the target protein substance and the target protein substance in a (n-1)-th training process of the initial prediction model; and
correcting a model parameter of the initial prediction model according to the binding strength sample qₙ and the binding strength sample qₙ₋₁ to obtain the protein prediction model.

11. The method according to claim 10, wherein a quantity of the similar protein fragment sample is at least two, and the at least two similar protein fragment samples respectively correspond to at least two antibody protein substance samples kₙ; and
the acquiring the binding strength sample qₙ between the antibody protein substance sample kₙ and the target protein substance comprises:
acquiring, for each of the at least two antibody protein substance samples kₙ, a target binding strength between the antibody protein substance sample kₙ and the target protein substance; and
determining an average strength of target binding strengths corresponding to the at least two antibody protein substance samples kₙ as the binding strength sample qₙ.

12. The method according to claim 10, wherein the correcting the model parameter of the initial prediction model according to the binding strength sample qₙ and the binding strength sample qₙ₋₁ to obtain the protein prediction model comprises:
acquiring a squared difference between the binding strength sample qₙ and the binding strength sample ^{q}n-1;
determining an excitation parameter for the initial prediction model according to the squared difference; and
correcting the model parameter of the initial prediction model according to the excitation parameter to obtain the protein prediction model.

13. A data processing apparatus, comprising:
an attribute acquiring module, configured to acquire protein attribute information of a reference protein substance, the reference protein substance comprising a protein adjusting region;
a predicted fragment generating module, configured to generate, by using the protein prediction model, a predicted protein fragment at the protein adjusting region in the reference protein substance according to the protein attribute information, the protein prediction model being obtained by training based on a target protein substance, and the protein prediction model being configured to predict a protein substance for binding to a target protein substance;
a fragment matching module, configured to identify a similar protein fragment matching the predicted protein fragment from a protein fragment database;
a substance synthesizing module, configured to perform virtual synthesis on the similar protein fragment and the reference protein substance to obtain synthetic substance auxiliary information, the synthetic substance auxiliary information being used for assisting in generation of an antibody protein substance for binding to the target protein substance.

14. A computer device, comprising a memory and a processor, the memory storing a computer program, the computer program, when executed by the processor, causing the processor to perform operations of the method according to any one of claims 1 to 12.

15. A computer-readable storage medium, the computer-readable storage medium storing a computer program, and the computer program being adapted to be loaded and executed by a processor to implement the method according to any one of claims 1 to 12.

16. A computer program product, comprising instructions, the instructions, when run on a computer, causing the computer to perform the method according to any one of claims 1 to 12.
